(19) Europäisches Patentamt
European Patent Office
Office européen des brevets

(11) Numéro de publication: **0 154 581**
**B1**

# (12) FASCICULE DE BREVET EUROPÉEN

(45) Date de publication du fascicule du brevet: **08.06.88**

(51) Int. Cl.⁴: **C 07 C 69/96**, C 07 C 68/02

(21) Numéro de dépôt: **85400251.6**

(22) Date de dépôt: **14.02.85**

(54) **Nouveaux carbonates alpha-chlorés, leur procédé de fabrication et leur application à la protection des fonctions amines des amino-acides.**

(30) Priorité: **16.02.84 FR 8402328**

(43) Date de publication de la demande: **11.09.85 Bulletin 85/37**

(45) Mention de la délivrance du brevet: **08.06.88 Bulletin 88/23**

(84) Etats contractants désignés: **AT BE CH DE FR GB IT LI LU NL SE**

(56) Documents cité: **DE-B-121 223**

(73) Titulaire: **SOCIETE NATIONALE DES POUDRES ET EXPLOSIFS, 12, quai Henri IV, F-75181 Paris Cédex 04 (FR)**

(72) Inventeur: **Barcelo, Gérard, 3, rue Mozart Résidence Claire- joie, F-91700 Sainte- Geneviève-des- Bois (FR)**
Inventeur: **Senet, Jean- Pierre, 79, rue de la Gare Herbeauvilliers- Buthiers, F-77760 La Chapelle- la-Reine (FR)**
Inventeur: **Sennyey, Gérard, 1, Place des 4-Pavés Saint- Aubin, F-91190 Gif- sur- Yvette (FR)**

**EP 0 154 581 B1**

Il est rappelé que: Dans un délai de neuf mois à compter de la date de publication de la mention de la délivrance du brevet européen toute personne peut faire opposition au brevet européen délivré, auprès de l'Office européen des brevets. L'opposition doit être formée par écrit et motivée. Elle n'est réputée formée qu'après paiement de la taxe d'opposition (Art. 99(1) Convention sur le brevet européen).

LIBER, STOCKHOLM 1988

**Description**

L'invention concerne de nouveaux carbonates α-chlorés, leur procédé de fabrication et leur application à la protection des fonctions amine des amino-acides.

Les carbonates sont des composés très appréciés, qui sont utilisés dans des domaines très variés par exemple comme solvants, plastifiants, lubrifiants, agents de transestérification ou intermédiaires dans la fabrication de peptides.

Pour réaliser certaines synthèses dans lesquelles interviennent les amino-acides, il est nécessaire de protéger temporairement la fonction amine de ces acides. Cette fonction amine est très souvent bloquée par transformation en fonction carbamte qui permet d'éviter le phénomène de racémisation lors du couplage et elle est facilement scindée, par exemple par acidolyse, hydrogénolyse ou tout autre méthode connue (E. Schröder et K. Lübke, "The Peptides", tome 1, page 39, Academic Press, New-York - Londres 1965).

Les groupements les plus utilisés à cet effet sont:

- le benzyloxycarbonyl (Z) ,
- le tertiobutyloxycarbonyl (BOC),
- le fluorénylméthyloxycarbonyl (FMOC),
- le trichloroéthyloxycarbonyl (TROC),
- le vinyloxycarbonyl (VOC)

Le groupement tertiobutyloxycarbonyl (BOC) est particulièrement apprécié.

L'introduction de ces groupements protecteurs s'effectue généralement par action du chloroformiate correspondant sur la fonction amine.

Cependant les chloroformiates ne sont pas toujours utilisables. Certains sont peu stables ou difficiles à manipuler. C'est le cas par exemple du chloroformiate de p-méthoxybenzyle, de furfuryle ou de tertiobutyle. Ce dernier, par exemple, même préparé in situ ne donne pas satisfaction du fait de la formation d'urées en présence d'un excès de phosgène.

D'autres agents de carbamatation ont été proposés tel que:

- les azides des différents groupes protecteurs. Leur synthèse s'effectue en plusieurs étapes et est délicate. Ils peuvent se décomposer de façon explosive, comme l'azide de BOC;
- les fluorures de BOC, de p-méthoxybenzyle ou de furfuryle mais leur préparation est difficile parce qu'elle impose le recours à des matières premières telles que ClCOF ou BrCOF, non commerciales, qui doivent être manipulées avec précaution;
- quelques essais ont été effectués avec des carbonates très particuliers tels que les carbonates mixtes des groupes protecteurs et de p-nitrophényle mais ils ne réagissent pas avec tous les acides aminés. Il se forme un alcool ou un phénol qui est très souvent difficile à éliminer et la réaction est réversible;
- des dicarbonates des groupes protecteurs. Leur synthèse est délicate et coûteuse. De plus, un reste protecteur est perdu;
- les carbonates d'oxime, d'hydroxysuccinimide, d'énol ou de S-diméthylpyrimidyle qui sont également difficile à préparer et nécessitent des matières premières coûteuses.

Il existe par conséquent depuis de nombreuses années un besoin non satisfait de composés nouveaux, faciles à fabriquer avec de bons rendements, stables, qui pourront être utilisés pour fixer sans difficulté les groupes protecteurs les plus intéressants sur la fonction amine des amino-acides et qui ne présentent pas les inconvénients des composés précités.

Selon l'invention les nouveaux carbonates alpha-chlorés ont pour formule générale

$R^1$ - O -C - O - CH - $CX_3$
‖ |
O Cl

dans laquelle X représente un atome de fluor, de chlore ou de brome et $R^1$ est différent du groupe

-CH - $CCl_3$ et représente :
|
Cl

un radical substitué ou non, saturé ou non, aliphatique en $C_11$ à $C_{20}$ ou araliphatique en $C_7$ à $C_{20}$, primaire, secondaire ou tertiaire, ou cycloaliphatique en $C_7$ à $C_{20}$, les substituants de $R^1$ étant choisis parmi les atomes d'halogène, les groupes comprenant du silicium, les groupes hétérocycliques ou non comprenant des atomes d'azote, d'oxygène ou de soufre.

De préférence X représente le chlore.

En particulier $R^1$ est choisi parmi les groupements habituellement utilisés pour protéger, sous forme de carbamates, la fonction amine des amino-acides tel que le tertiobutyle, le paranitrobenzyle, le 9-fluorénylméthyle, le trichloro-2,2,2 éthyle, le triméthylsilyléthyle.

Selon l'invention les nouveaux carbonates alpha-chlorés sont preparés par réaction d'un composé hydroxylé de formule $R^1OH$ sur un chloroformiate alphachloré de formule:

$CX_3$ -CH - O - C - Cl
  |  ||
  Cl  O

X et $R^1$ ayant les significations précédentes, dans un milieu solvant à une température de -20° à +50°C en présence d'un agent accepteur d'acide chlorhydriqe que l'on ajoute à la suite des deux réactifs.

Il se produit la réaction suivante:

$R^1$OH + Cl -C - O - CH - $CX_3$ → $R^1$ - OC - O CH - $CX_3$ + HCl
  ||  |  ||  |
  O  Cl  O  Cl

Le milieu solvant est constitué d'un ou de plusieurs solvants inertes vis-à-vis des réactifs. On choisit de préférence les solvants aliphatiques chlorés par exemple le dichlorométhane ou le dichloroéthane, les éthers cycliques ou non, les cétones telles que l'acétone ou la butanone-2, les nitriles, les esters ou les hydrocarbures aliphatiques ou aromatiques.

L'agent accepteur d'acide est en général une base organique ou minérale. De préférence on utilise la pyridine ou la triéthylamine.

On ajoute la base à la suite des deux composants réactifs, et de préférence progressivement.

La température est de préférence comprise entre -5° et +5°. On peut à la fin de la réaction porter le mélange pendant quelques minutes à quelques heures à la température ambiante.

Les chloroformiates α-chlorés eux-mêmes peuvent être préparés par chloration des chloroformiates ou de préférence par phosgénation des aldéhydes correspondants comme décrit dans la demande européenne n° 40153.

Les composés de départ et la base sont généralement introduits en quantité stoechiométrique. On peut utiliser un excès de l'alcool. Le carbonate obtenu est facilement isolé par les méthodes usuelles.

Les carbonates de l'invention n'ont jamais été jusqu'à présent décrits dans la littérature. La présence dans leur structure d'un groupement

$CX_3$ -CH -
  |
  Cl

leur donne une grande originalité et des propriétés très spécifiques qui les rendent très utiles comme intermédiaires de synthèse.

L'invention concerne en particulier une application des nouveaux carbonates α -chlorés.

Dans cette application les nouveaux carbonates α -chlorés précédemment décrits sont utilisés pour protéger la fonction amine des amino-acides.

Plus précisément ils sont mis à réagir, dans un milieu solvant en présence d'un agent de fixation d'acide chlorhydrique et à une température comprise entre -5° et 100°C avec un acide amino et/ou imino carboxylique comportant au moins un atome d'hydrogène sur le groupe amino ou imino, de formule

NH - $R^3$ - COOH
|
$R^4$

dans laquelle $R^3$ et $R^4$
indépendamment ou reliés représentent les radicaux habituels des amino-acides.

La réaction peut s'écrire:

$R^1$ O -C - O - CH - $CX_3$ + H - N - $R^3$ - COOH
  ||  |  |
  O  Cl  $R^4$

→ $R^1$ - O - C - N - $R^3$ - COOH + $CX_3$CHO + HCl
  ||  |
  O  $R^4$

Cette réaction est surprenante, il y a en effet élimination de HCl mais pas avec fixation du reste aminé sur le carbone porteur du chlore pour former:

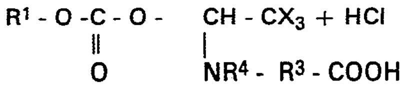

comme on pouvait normalement s'y attendre et comme c'est le cas par exemple lors de la réaction d'un α -chloré sur un acide:

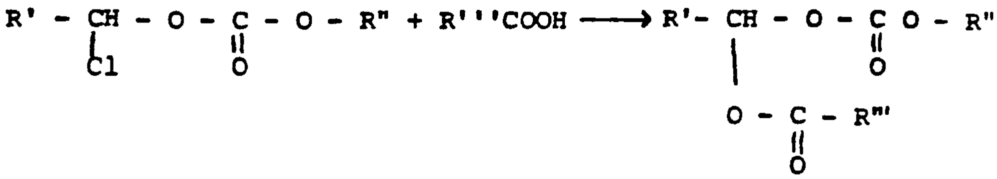

(ASTRA - Brevet FR 2 201 870)

Au contraire les nouveaux composés α-chlorés se coupent, le reste

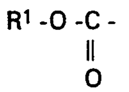

se fixe sur l'azote de l'amino-acide et il se forme l'aldéhyde $CX_3CHO$.

Comme amino-acides ou imino-acides carboxyliques de départ on peut utiliser tous les composés naturels ou synthétiques, optiquement actifs ou non, ou racémiques qui contiennent encore au moins un atome d'hydrogène fixé sur le groupe amino ou imino.

Le radical $R^3$ ou $R^4$ peut contenir des groupes fonctionnels tels que amino, imino, mercapto, hydroxyle ou carboxyle, bloqués ou non.

Le radical $R^4$ représente généralement un atome d'hydrogène et $R^3$, un radical aliphatique en $C_1$ à $C_{20}$ ou araliphatique en $C_7$ à $C_{20}$, substitué ou non, saturé ou non.

L'hétérocycle formé par $R^3$ et $R^4$ et l'atome d'azote peut avoir de 4 à 6 chaînons, il peut être saturé ou insaturé, substitué ou non, condensé ou non à d'autres cycles par exemple aromatiques.

L'acide peut être sous forme d'un de ses dérivés par exemple un ester ou un amide.

L'invention s'applique le plus souvent à des alpha amino-acides mais des bêta, gamma, et delta amino-acides peuvent également être utilisés.

Le groupe carboxylique peut aussi être remplacé par un groupe sulfonique ou phosphorique.

A titre d'exemple d'amino-acides, on peut citer la L-phénylalanine, la L-proline, la glycine, la L-tyrosine, la L-sérine, l'acide L-aspartique, le glycinate d'éthyle, la phénylglycine, la L-alanine.

Comme carbonates α -chlorés on utilise de préférence ceux dans lesquels X est le chlore et le radical $R^1$, un des groupes protecteurs les plus couramment utilisés pour acyler la fonction amine, par exemple les carbonates de tétrachloro- 1,2,2,2 éthyle et de trichlo-ro-2',2',2' éthyle, de 9-fluorényl méthyle, de paranitrobenzyle ou de 2-triméthylsilyl éthyle. Ces carbonates sont notamment très appréciés dans le cas d'hydroxy amino-acides non protégés tel que la L-sérine ou la L-tyrosine. Le carbonate de tétrachloro- 1,2,2,2 éthyle et de tertiobutyle est particulièrement intéressant.

La présence d'un agent de fixation d'acide est nécessaire pour éliminer l'acide chlorhydrique qui se forme au cours de la réaction. Ce peut être une base organique ou minérale.

Parmi les bases préférées on peut citer. l'hydroxyde de sodium ou de potassium, le carbonate ou le bicarbonate de sodium ou de potassium, l'oxyde de magnésium qui sont généralement mis en oeuvre sous forme de solution aqueuse, les amines tertiaires, par exemple, la pyridine et la triéthylamine.

On introduit le plus souvent la substance basique en excès, de préférence de l'ordre de 1,1 à 3 équivalents par fonction amine à protéger.

Il peut être avantageux de maintenir le PH constant durant toute la réaction à l'aide d'appareils classiques.

Le milieu solvant peut être constitué d'un ou de plusieurs solvants inertes vis-à-vis des réactifs. Comme solvant organique on choisit de préférence des solvants aliphatiques chlorés tels que le dichlorométhane ou le dichloro-1,2 éthane, les éthers cycliques ou non, par exemple, le tétrahydrofuranne ou le dioxanne, l'acétone, la pyridine, l'acétonitrile, le diméthylformamide, les alcools tels que l'éthanol ou le tertiobutanol. L'eau peut également être utilisée seule ou en mélange avec les solvants précités. Le mélange dioxanne-eau 1/1 est particulièrement apprécié.

La température de réaction dépend de la nature du solvant, de la réactivité des composés de départ ainsi que des autres conditions de la réaction. Elle est de préférence comprise entre 0° et 30° C.

La pression atmosphérique est la pression la plus couramment utilisée. Le temps de réaction est variable. Il est généralement compris entre 0,5 et 36 heures, le plus souvent entre 2 et 6 heures.

Les composés de départ peuvent être ajoutés en quantité stoechiométrique. Le plus souvent on préfère utiliser un excès d'un des réactifs.

L'ordre d'introduction des réactifs n'est pas une caractéristique fondamentale de l'invention. Généralement on additionne le carbonate α -chloré à la suite de l'acide aminé.

Les amino-acides bloqués peuvent être facilement récupérés et isolés sous forme cristallisée éventuellement en les transformant en sel d'ammonium, par exemple en sel de dicyclohexylammonium.

Grâce aux nouveaux carbonates α-chlorés de l'invention obtenus à partir de matières premières disponibles, et par un procédé simple, on peut effectuer le blocage sous forme de carbamates de la fonction amine d'amino-acides très variés par tous les groupes protecteurs connus. Jusqu'à présent cela était soit impossible, soit très difficile à réaliser. Les rendements sont très élevés. Les conditions de réaction sont douces et il ne se produit pas de racémisation.

Sur les amino-acides ainsi protégés on peut effectuer toutes les opérations de couplage souhaité de la fonction acide, classiques en synthèse peptidique (cf. par exemple, E. Gross & J. Meienhofer Eds, "The peptides. Analysis, synthesis, biology" Academic Press, New York & London Vol. 3, 1980).

Ces dérivés des amino-acides sont très utilisés comme produits intermédiaires pour la fabrication de produits alimentaires et pharmaceutiques. Par exemple on peut citer la synthèse de l'ASPARTAM (cf. Tetrahedron, Vol. 39 n° 24, p. 4121 à 4126, 1983. B. YDE & Al).

L'invention est illustrée par les exemples qui suivent.

**Exemple 1**

Synthèse du carbonate de tétrachloro- 1,2,2,2 éthyle et de tertiobutyle

On ajoute en une seule fois 9,9 g (0,04 mole) de chloroformiate de tétrachloro- 1,2,2,2 éthyle à une solution de tertiobutanol (3 g; 0,04 mole) dans le dichlorométhane (50 ml). On refroidit à 0°C et on ajoute goutte à goutte 3,2 g (0,04 mole) de pyridine. On agite 4 heures à température ambiante. On ajoute alors 20 ml d'eau glacée, on sépare la phase organique et on lave avec 20 ml d'eau glacée. On sèche sur sulfate de magnésium et on évapore le solvant. On obtient 11,3 g d'un solide blanc (rendement 99 %) que l'on recristallise dans l'éther de pétrole (rendement 87 % ; Pt F : 70°C), et l'on obtient 9,9 g du carbonate purifiée.

Pt Eb 96°C/ 866 Pa(6,5 mm Hg)

IR $\nu$CO = 1770 $cm^{-1}$

RMN $H^1$ ($CDCl_3$, TMS) : 1,5 (s, $CH_3$) 6,7(s,CH)

**Exemple 2**

Préparation de la tertiobutyloxycarbonyl-L-phénylalanine

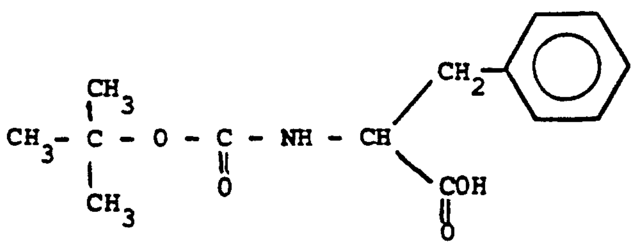

A une solution de L-phénylalanine (1,65 g ; 10 mmole) dans le dioxanne aqueux (1 : 1 ; 30 ml). On ajoute 4,2 ml (30 mmoles) de triéthylamine et on agite jusqu'à dissolution (environ 10 mn). On ajoute alors 2,85 g (10 mmoles) de carbonate de tertiobutyle et de tétrachloro-1,2,2,2 éthyle et on agite 6 heures à 20° C. On ajoute alors 50 ml d'eau et on extrait avec 2 x 20 ml d'acétate d'éthyle. La phase aqueuse est acidifiée (pH 2-3) avec HCl N puis extraite avec 3 x 30 ml d'acétate d'éthyle. L'extrait est lavé avec une solution saturée de NaCl, séché sur $MgSO_4$ et évaporé. LA produit obtenu est cristallisé dans l'acétate d'éthyle et l'éther de pétrole. On obtient 2,1 g du carbamate attendu (rendement 79 %) ; Pt F : 85-87°C ;

Pt $F_{Litt}$ = 86-88°C, pouvoir rotatoire = + 28 (c 1,5 EtOH);
Litt = + 24,7 (c 1,5 EtOH)

b) Préparation de la N-tertiobutyloxycarbonyl-L-alanine

On procède comme à l'exemple 2 a).
A partir de 1,78 g (20 mmoles) d'alanine, on obtient 3,4 g (Rendement : 90 %) de BOC-L-alanine.
Pt F = 80-81°C ; Pt $F_{Litt}$ = 83-84°C

$[\alpha]_D^{20}$ = - 24,9 (c 2,1 AcOH)    $[\alpha]_D^{20}$ = 22,4 (c 2,1 AcOH)

**Exemple 3**

Préparation de la tertiobutyloxycarbonyl-L-proline

On opère comme dans l'exemple 2. A partir de 1,15 g (10 mmoles) de Lproline, on obtient 1,95 g (rendement 91 %) du carbamate attendu.

Pt F = 130-131° C ; Pt $F_{Litt}$ = 132-133° C.

$[\alpha]_D^{20}$ = -60 (c 2,0 AcOH)    $[\alpha]_D^{20}$ = -60,2 (c 2,0 AcOH)

**Exemple 4**

Préparation de la tertiobutyloxycarbonyl-glycine (BOC-Gly)

On opère comme dans l'exemple 2. A partir de 0,75 g (10 mmoles) de glycine, on obtient 1,5 g (rendement 86 %) du carbamate attendu.

Pt F = 80-85° C ; Pt $F_{Litt}$ = 86-88° C

**Exemple 5**

a) Préparation de BOC-Gly à PH contrôlé

On dissout 5,6 g (0,075 mole) de glycine dans 150 ml de dioxanne aqueux (50%) et on ajuste le PH avec de la soude 4N. On ajoute en une fois 23,6 g (0,083 mole) du carbonate de tétrachloro- 1,2,2,2 éthyle et de tertiobutyle et on maintient le pH constant par addition de soude 4N. Lorsque la réaction est terminée on rajoute environ 200 ml d'eau puis on lave la phase aqueuse avec 2 fois 100 ml d'éther éthylique. On acidifie alors la phase aqueuse pH3 avec HCl 6N et on extrait avec 3 fois 200 ml d'acétate d'éthyle (AcOEt). Après séchage et évaporation du solvant, on cristallise dans AcOEther de pétrole (40-70° C). On obtient le BOC-glycine qui fond à 85-87° C.

| PH | durée | soude consommée | rendement isolé |
|---|---|---|---|
| 10 | 5 h | 2 eq | 45 % |
| 9 | 20 h | 2 eq | 45 % |
| 8 | 30 h | 1 eq | 31 % |

b) On procède comme en a) en utilisant divers acides aminés.

Les résultats sont rassemblés dans le tableau suivant:

| Acide aminé | pH | Rdt % | Pt F °C | $[\alpha]_D^{20}$ | c/solvant |
|---|---|---|---|---|---|
| Pro | 8,6 | 80 | 135-136 | -60 | 2/AcOH |
| Trp | 8,3 | 74 | 135-140 | -21,2 | 1/AcOH |
| Asp (OBzl) | * | 42 | 98-100 | -19 | 2/DMF |
| His(Tos) | 10 | 50 | 154 | + 25,4 | 1/MeOH |
| Ala | 10,1 | 70 | 84 | - 25,5 | 2/AcOH |
| Val | 9,5 | 80 | 78 | - 6,0 | 1/AcOH |
| Ile | 9,5 | 71 | 66 | + 3,3 | 1/AcOH |
| Leu | 9,75 | 95 | 75 | -27 | 1/AcOH |
| Met | 9,7 | 75 | huile | - | - |
| Glu (OBzl) | 9 | 42 | 132 (sel DCHA) | + 13,6 | 1,1/MeOH |
| His (Tos) | * | 50 | 154 | + 25,4 | 1/MeOH |
| Tyr (Bzl) | 9,8 | 43 | 98-100 | + 27,4 | 2/EtOH |
| Ser (Bzl) | 9,5 | 74 | 61 | + 21 | 2/EtOH |
| Thr (Bzl) | 9,0 | 76 | 114 | + 16,3 | 1/MeOH |
| Arg $NO_2$ | 9,5 | 25 | 100-114 | - 23,0 | 1,9/pyridine |
| Cys (Acm) | 9,5 | 46 | - | - | - |
| Lys (z) | 10,2 | 84 | huile | - | - |

* base utilisée : triéthylamine

**Exemple 6**

Préparation de la tertiobutyloxycarbonyl-L-tyrosine

On dissout 1,81 g (10 mmole) de tyrosine dans 20 ml de dioxanne aqueux (1 : 1) par addition de 1,4 ml (10 mmoles) de triéthylamine et 15 mmoles de soude. On ajoute alors 2,85 g (10 mmoles) de carbonate de tertiobutyle et de tétrachloro-1,2,2,2 éthyle, et on agite 6 heures à 20°C.

On opère ensuite comme dans l'exemple 2.

Le produit obtenu est cristallisé sous forme de sel de dicyclohexylammonium. On obtient 3,8 g (rendement 82 %).

Pt F = 206°C ; Pt $F_{Litt}$ = 212°C.

**Exemple 7**

Préparation de la tertiobutyloxycarbonyl-L-serine

On opère comme dans l'exemple 2, la durée de la réaction étant de 24 heures au lieu de 6 heures. A partir de 1,05 g (10 mmoles) de L-Serine on obtient 3,1 g (rendement 78%) du carbamate souhaité sous forme de sel de dicyclohexyl ammonium.

Pt F = 139 - 140°C ; Pt $F_{Litt}$ = 140-142°C

$[\alpha]_D^{20}$ = +8 (c 2,8 MeOH) ; $[\alpha]_{D\,Litt}^{20}$ = +13 (c = 3,0 MeOH

**Exemple 8**

Préparation de l'acide tertiobutyloxycarbonyl-L-aspertique

On opère comme dans l'exemple précèdent. A partir de 1,33 g (10 mmoles) d'acide L-aspartique, on obtient 1,4 g (rendement 60 %) de l'acide attendu.

Pt F = 116 - 118°C ; Pt $F_{Litt}$ = 114 - 116°C.

$[\alpha]_D^{20}$ = -5(c 1,0 MeOH) ; $[\alpha]_D^{20}$ $_{Litt}$ = -6,2(c 1,0 MeOH)

**Exemple 9**

Préparation du carbonate de 1,2,2,2-tétrachloroéthyle et 9-fluorénylméthyle.

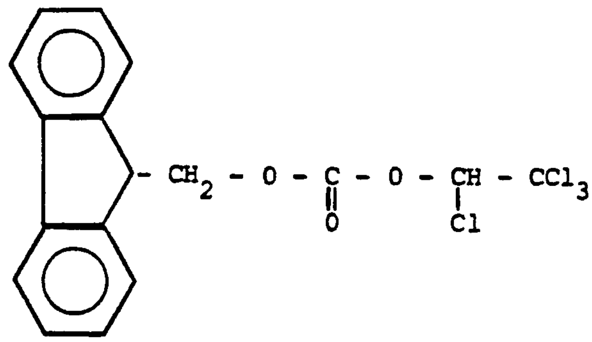

On ajoute en une seule fois 6,7 g (0,027 mole) de chloroformiate de 1,2,2,2 tétrachloroéthyle à une solution de 9-fluorénylméthanol (4,9 g ; 0,025 mole) dans 50 ml de dichlorométhane.

On refroidit à 0° C et on ajoute goutte à goutte 2,2 ml de pyridine. On agite 4 heures à 0° C. On ajoute alors 50 ml de dichlorométhane et on lave la phase organique avec 2 fois 50 ml d'eau glacée. On sèche sur sulfate de magnésium et on évapore le solvant. On cristallise le résidu dans l'hexane, on obtient 9,3 g du carbonate attendu (rendement 98 %) ;

Pt F = 98-100° C

I R :$\gamma$C = 0 1750 $cm^{-1}$

RMN $H^1$ : ($CDCl_3$, TMS) 4,5 ppm $CH_2$ -0 ; 6,75 ppm CH - Cl
|
O

**Exemple 10**

Préparation de la 9-fluorénylméthyloxycarbonyl-L-phénylalanine

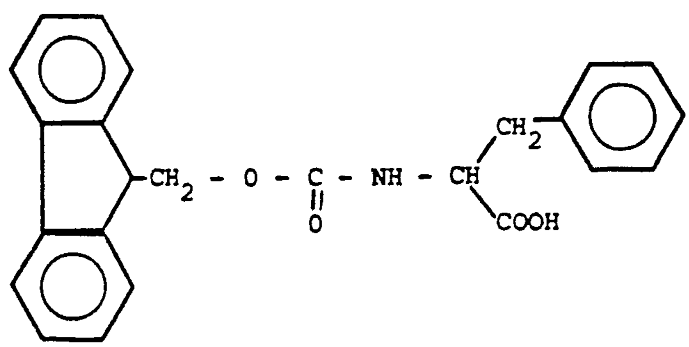

On dissout 0,83 g de L-phenylalanine (5 mmoles) dans le dioxanne aqueux (1 : 2 ; 12 ml) contenant 1,4 ml de triéthylamine (10 mmoles). On refroidit à 0° C et on ajoute en une fois 2,05 g (5 mmoles) du carbonate précédent dissout dans 4 ml de dioxanne. Après 2 heures à 0° C on ajoute 20 ml d'eau et on extrait avec 2 fois 20 ml d'éther. On acidifie alors la phase aqueuse (PH-2-3) avec HCl 6N et on extrait avec 3 fois 50 ml d'acétate d'éthyle. On sèche sur $MgSO_4$ et on évapore. Le produit obtenu cristallise dans l'acétate d'éthyle et l'éther de pétrole, on obtient 1,44 g du dérivé attendu (rendement 75 %).

Pt F = 178-179° C

Pt $F_{Litt}$ = 178-179° C (Litt : L-Lapatsanis et al. Synthesis (1983) 671).

**Exemple 11**

Préparation de la 9-fluorénylméthyloxycarbonyl (L)-proline (FMOC-Pro).

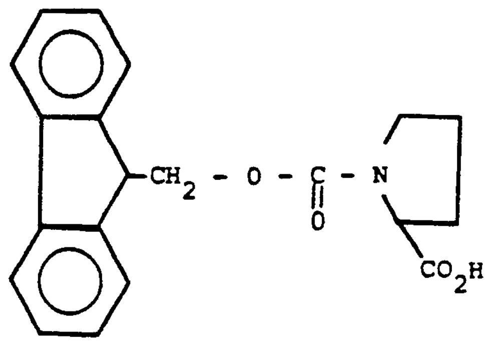

On procède comme à l'exemple 10. A partir de 0,58 g (5 mmoles) de L-Proline on obtient 1,4 g (rendement 83 %) de FMOC-L-Proline.
Pt F = 112-113°C (Pt $F_{Litt}$ = 116-117°C)

**Exemple 12**

Préparation de la 9-fluorénylméthyloxycarbonyl-L-serine.

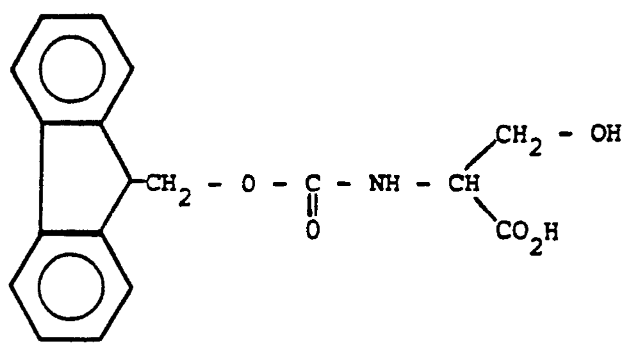

On procède comme à l'exemple 10 mais la réaction est poursuivie 24 heures à 20°C. A partir de 0,53 g (5 mmoles) de L-Serine on obtient 1,32 g (rendement 81%) de FMOC-(L)-Serine, PtF = 73-75°C. Après recristallisation PtF = 83-86°C ($PtF_{Litt}$ = 86-88°C)

**Exemple 13**

Préparation du carbonate de trichloro-2,2,2 éthyle et de tétrachloro-1',2',2',2' éthyle.

$Cl_3C - CH_2 - O - C(=O) - O - CH(Cl) - CCl_3$

On procède comme dans l'exemple 1. A partir de 14,9 g de trichloroéthanol (0,1 mole) on obtient 24,1 g (rendement 67 %) du carbonate attendu.
Pt Eb = 108°C/6,6 Pa ; Pt F = 36°C
I R $\nu$CO = 1770 $cm^{-1}$
RMN $H^1$ ($CDCl_3$, TMS) : 4,85 (s, $CH_2$) 6,7 (s, CH)

**Exemple 14**

Préparation de trichloroéthoxycarbonyl-L-phénylalanine (TROC-L-Phe)

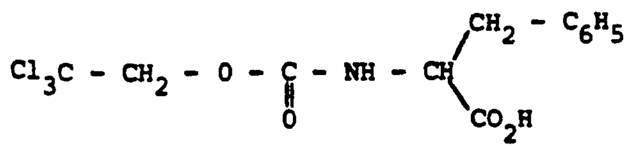

On procède comme dans l'exemple 10. A partir de 0,83 g (5 mmoles) de L-phénylalanine et 1,98 g (5,5 mmoles) de carbonate de trichloro-2,2,2 éthyle et de tétrachloro-1',2',2',2' éthyle, on obtient 1,43 g (rendement 84 %) de TROC-L-Phénylalanine.

Pt F = 128-129° C (Pt $F_{Litt}$ = 129-130° C)

**Exemple 15**

Préparation de trichloroéthoxycarbonyl-(L)-Serine.

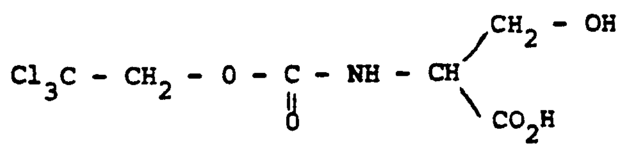

On procède comme dans l'exemple 12. A partir de 0,53 g (5 mmoles) de L-serine et 1,98 g (5,5 mmoles) du carbonate de l'exemple 13, on obtient 1,15 g (rendement 82 %) de TROC-L-Serine.

Pt F = 111-113° C ; Pt $F_{Litt}$ = 114-115° C.

**Exemple 16**

Préparation du carbonate de tétrachlor-1,2,2,2 éthyle et 2-triméthylsilyléthyle.

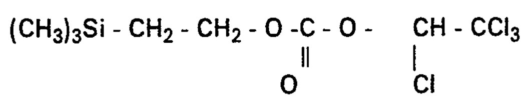

On procède comme dans l'exemple 1. A partir de 5,91 g de triméthyl silyléthanol et 12,35 g de chloroformiate de tétrachloroéthyle on obtient 13,6 g (rendement 83 %) de produit attendu ; E = 92-94° C/6,6 Pa

I R $\gamma$CO = 1750 $cm^{-1}$ RMN $H^1$ ($CDCl_3$, TMS externe) = 0,1 (s, $CH_3$-Si) 1,1 (t, $CH_2$-Si) 4,35 (t, $CH_2$-0) 6,7 (s, CH-Cl)

**Exemple 17**

Préparation de la triméthylsilyléthyloxycarbonyl-(L)-phénylalanine.

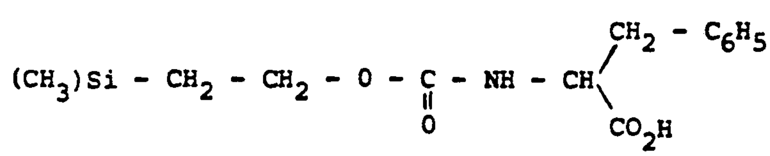

On procède comme dans l'exemple 12. A partir de 0,83 g (5 mmoles) de phénylalanine et 1,8 g (5,5 mmoles) du carbonate précédent, on obtient 1,4 g (rendement 100 %) de triméthylsilyléthoxycarbonyl-L-phénylalanine sous forme d'une huile.

RMN $H^1$ ($CDCl_3$, TMS) O(s,$CH_3$-Si) 0,9 (t, $CH_2$-Si) 3,0($CH_2$Ph)

```
4,0(t,O-CH2-C-Si) 4,5 (m,CH - N) 5,2 (s,NH) 7,2 (s,Ph-H) 8,7(C -OH)
                         |                                    ||
                        CO2                                   O
```

On ajoute 2 ml de dicyclohexylamine à cette huile dissoute dans 5 ml d'éther et on recueille après cristallisation 1,93 g (rendement 78 %) de sel de dicyclohexylammonium.

Pt F = 111-112°C.

**Exemple 18**

<u>Synthèse du carbonate de p-nitrobenzyle et 1,2,2,2-tétrachloroéthyle</u>

On dissout 3,83 g (25 mmoles) d'alcool p-nitrobenzylique et 6,17 g (25 mmoles) de chloroformiate de 1,2,2,2-tétrachloroéthyle dans 50 ml de dichlorométhane. On refroidit à 0°C et on ajoute goutte à goutte 2,02 ml de pyridine. On agite 4 heures à 10°C puis on ajoute 50 ml d'eau glacée, on sépare la phase organique et on lave encore avec deux fois 50 ml d'eau. On sèche la phase organique sur sulfate de magnésium, on évapore le solvant et on obtient 8,7 g du produit souhaité (Rendement : 96 %).

Pt Eb = 190 - 195°C / 0,05 mm Hg.

Pt F = 53 - 55°C (Solvant de cristallisation : éthanol aqueux ; rendement de cristallisation : 54 %).

**Revendications** pour les Etats contractants: BE CH DE FR GB IT LI LU NL SE

1. Nouveaux carbonates alpha-chlorés caractérisés en ce qu'ils ont la formule :

```
R1 - O - C- O -   CH - CX3
         ||       |
         O        Cl
```

dans laquelle X représente un atome de fluor, de chlore ou de brome et $R^1$ est différent du groupe

```
-     CH - CCl3
           |
           Cl
```

et représente:
un radical substitué ou non, saturé ou non, aliphatique en $C_1$ à $C_{20}$ ou araliphatique en $C_7$ à $C_{20}$, primaire, secondaire ou tertiaire, ou cycloaliphatique en $C_7$ à $C_{20}$, les substituants de $R_1$ étant choisis parmi les atomes d'halogène, les groupes comprenant du silicium, les groupes hétérocycliques ou non comprenant des atomes d'azote, d'oxygène ou de soufre.

2. Carbonates alpha-chlorés selon la revendication 1 caractérisés en ce que X est le chlore.

3. Carbonates alpha-chlorés selon l'une quelconque des revendications précédentes caractérisés en ce que $R^1$ est le groupe tertiobutyle, 9-fluorénylméthyle, trichloro-2,2,2 éthyle, triméthylsilyléthyle, paranitrobenzyle.

4. Carbonate alpha-chloré selon l'une quelconque des revendications précédentes caractérisé en ce que c'est le carbonate de tétrachloro-1,2,2,2 éthyle et de tertiobutyle.

5. Procédé de préparation des carbonates alpha-chlorés selon l'une quelconque des revendications précédentes caractérisé en ce qu'on fait réagir un composé de formule $R^1OH$ sur un chloroformiate alpha-chloré de formule:

```
CX3 -CH - O -  C - Cl
     |         ||
     Cl        O
```

X et $R^1$ ayant les significations précédentes, dans un milieu solvant à une température de -20° à + 50°C en présence d'un agent acccepteur d'acide que l'on ajoute à la suite des deux composés de départ.

6. Procédé selon la revendication 5 caractérisé en ce que l'agent accepteur d'acide est une base organique ou minérale.

7. Procédé selon la revendication 6 caractérisé en ce que la base est ajoutée progressivement.

8. Procédé selon la revendication 6 ou 7 caractérisé en ce que la base est la pyridine ou la triéthylamine.

9. Procédé selon l'une quelconque des revendications 5 à 8 caractérisé en ce que le milieu solvant est constitué d'un ou de plusieurs solvants inertes vis-à-vis des réactifs choisis parmi les solvants aliphatiques chlorés, les éthers cycliques ou non, les cétones, les nitriles, les esters et les hydrocarbures aliphatiques ou

aromatiques.

10. Utilisation des carbonates alpha-chlorés selon l'une quelconque des revendications 1 à 4 caractérisée en ce qu'on fait réagir le carbonate alpha-chloré dans un milieu solvant en présence d'un agent de fixation d'acide et à une température comprise entre -5° et 100°C avec un acide amino et/ou iminocarboxylique comportant au moins un atome d'hydrogène fixé sur le groupe amino ou imino, de formule

$$\underset{\displaystyle R^4}{\overset{|}{NH}} - R^3 - COOH$$

dans laquelle $R^3$ et $R^4$ indépendamment ou reliés représentent les radicaux habituels des amino-acides naturels ou synthétiques.

11. Utilisation selon la revendication 10 caractérisée en ce que l'amino-acide est un composé naturel ou synthétique, optiquement actif ou non, ou racémique.

12. Utilisation selon la revendication 10 ou 11 caractérisée en ce que $R^4$ représente un atome d'hydrogène, $R^3$ un radical aliphatique en $C_1$ à $C_{20}$ ou araliphatique en $C_7$ à $C_{20}$, substitué ou non, saturé ou non ; ou $R^3$ et $R^4$ sont reliés et forment avec l'atome d'azote un hétérocycle, avec 4 à 6 chaînons, saturé ou non, substitué ou non.

13. Utilisation selon l'une quelconque des revendications 10 à 12 caractérisée en ce que $R^3$ et/ou $R^4$ sont substitués par des groupes fonctionnels bloqués ou non.

14. Utilisation selon l'une quelconque des revendications 10 à 13 caractérisée en ce que le groupe carboxylique de l'amino-acide est remplacé par un groupe sulfonique ou phosphorique.

15. Utilisation selon l'une quelconque des revendications 10 à 14 caractérisée en ce que l'amino-acide est sous forme d'un dérivé de l'acide.

16. Utilisation selon l'une quelconque des revendications 10 à 15 caractérisée en ce que l'amino-acide est la L-phénylalanine, la L-proline, la glycine, la L-tyrosine, la L-sérine, l'acide L-aaspartique, le glycinate d'éthyle, la phénylglycine, la L-alanine, le tryptophane, l'ester benzylique de l'acide aspartique, l'histidine, le tosylate d'histidine, l'alanine, la valine, l'isoleucine, la leucine, la méthionine, l'ester benzylique de l'acide glutamique, l'éther benzylique de la tyrosine, de la sérine, de la thréonine, la nitroarginine, la benzyloxycarbonyl lysine, l'acétomidométhyle cystéïne.

17. Utilisation selon l'une quelconque des revendications 10 à 16 caractérisée en ce que le milieu solvant est constitué d'un ou de plusieurs solvants inertes vis-à-vis des réactifs choisis parmi les solvants aliphatiques chlorés, les éthers cycliques ou non, l'acétone, la pyridine, l'acétonitrile, le diméthylformamide et les alcools.

18. Utilisation selon la revendication 17 caractérisée en ce que le milieu solvant contient de l'eau.

19. Utilisation selon la revendication 18 caractérisée en ce que le milieu solvant est un mélange dioxanne-eau dans le rapport 1 : 1.

20. Utilisation selon l'une quelconque des revendications 10 à 19 caractérisée en ce que l'agent de fixation d'acide est une base organique ou minérale.

21. Utilisation selon la revendication 20 caractérisée en ce que la base est choisie parmi l'hydroxyde de sodium ou de potassium, le carbonate ou le bicarbonate de sodium ou de potassium, l'oxyde de magnésium, les amines tertiaires.

22. Utilisation selon la revendication 21 caractérisée en ce que l'amine tertiaire est la pyridine ou la triéthylamine.

23. Utilisation selon l'une quelconque des revendications 10 à 22 caractérisée en ce que la réaction est effectuée à une température comprise entre 0° et 30°C.

24. Utilisation selon l'une quelconque des revendications 10 à 23 caractérisée en ce que l'on maintient constante la valeur du pH.

**Revendications** pour l'Etat contractant: AT

1. Procédé de préparation des carbonates alpha-chlorés de formule:

$$R^1 - O - \underset{\displaystyle O}{\underset{\|}{C}} - O - \underset{\displaystyle Cl}{\underset{|}{CH}} - CX_3$$

dans laquellle X représente un atome de fluor, de chlore ou de brome et $R^1$ est différent du groupe

$- CH - CCl_3$
|
Cl

et représente:
un radical substitué ou non, saturé ou non, aliphatique en $C_1$ à $C_{20}$ ou araliphatique en $C_7$ à $C_{20}$, primaire, secondaire ou tertiaire, ou cycloaliphatique en $C_7$ à $C_{20}$, les substituants de $R_1$ étant choisis parmi les atomes d'halogène, les groupes comprenant du silicium, les groupes hétérocycliques ou non comprenant des atomes d'azote, d'oxygène ou de soufre, caractérisé en ce qu'on fait réagir un composé de formule $R^1OH$ sur un chloroformiate alpha-chloré de formule:

$CX_3 - CH - O - C - Cl$
|   ||
Cl   O

X et $R^1$ ayant les significations précédentes, dans un milieu solvant à une température de -20° à +50°C en présence d'un agent d'accepteur d'acide que l'on ajoute à la suite des deux composés de départ.

2. Procédé de préparation selon la revendication 1 caractérisé en ce que X est le chlore.

3. Procédé de préparation selon la revendication 1 ou 2 caractérisé en ce que $R^1$ est le groupe tertiobutyle, 9-fluorénylméthyle, trichloro-2,2,2 éthyle, triméthylsilyléthyle, paranitrobenzyle.

4. Procédé de préparation selon la revendication 1 caractérisé en ce que le carbonate préparé est le carbonate de tétrachloro-1,2,2,2 éthyle et de tertiobutyle.

5. Procédé selon l'une quelconque des revendications précédentes caractérisé en ce que l'agent accepteur d'acide est une base organique ou minérale.

6. Procédé selon la revendication 5 caractérisé en ce que la base est ajoutée progressivement.

7. Procédé selon la revendication 5 ou 6 caractérisé en ce que la base est la pyridine ou la triéthylamine.

8. Procédé selon l'une quelconque des revendications précédentes caractérisé en ce que le milieu solvant est constitué d'un ou de plusieurs solvants inertes vis-à-vis des réactifs choisis parmi les solvants aliphatiques chlorés, les éthers cycliques ou non, les cétones, les nitriles, les esters et les hydrocarbures aliphatiques ou aromatiques.

9. Utilisation des carbonates alpha-chlorés de formule:

$R^1 - O - C - O - CH - CX_3$
||   |
O   Cl

dans laquellle X représente un atome de fluor, de chlore ou de brome et $R^1$ est différent du groupe

$- CH - CCl_3$
|
Cl

et représente:
un radical substitué ou non, saturé ou non, aliphatique en $C_1$ à $C_{20}$ ou araliphatique en $C_7$ à $C_{20}$, primaire, secondaire ou tertiaire, ou cycloaliphatique en $C_7$ à $C_{20}$, les substituants de $R^1$ étant choisis parmi les atomes d'halogène, les groupes comprenant du silicium, les groupes hétérocycliques ou non comprenant des atomes d'azote, d'oxygène ou de soufre, caractérisée en ce qu'on fait réagir le carbonate alpha-chloré dans un milieu solvant en présence d'un agent de fixation d'acide et à une température comprise entre -5° et 100°C avec un acide amino et/ou iminocarboxylique comportant au moins un atome d'hydrogène fixé sur le groupe amino ou imino, de formule

$NH - R^3 - COOH$
|
$R^4$

dans laquelle $R^3$ et $R^4$ indépendamment ou reliés représentant les radicaux habituels des amino-acides naturels ou synthétiques.

10. Utilisation selon la revendication 9 caractérisée en ce que dans la formule des carbonates alpha-chlorés X représente le chlore.

11. Utilisation selon la revendication 9 ou 10 carctérisée en ce que le groupe $R^1$ des carbonates alpha-chlorés représente le groupe tertiobutyle, 9-fluorénylméthyle, trichloro-2,2,2 éthyle, triméthylsilyléthyle, paranitrobenzyle.

12. Utilisation selon la revendication 9 caractérisée en ce que le carbonate alpha-chloré est le carbonate de tétrachloro-1,2,2,2 éthyle et de tertiobutyle.

13. Utilisation selon l'une quelconque des revendications 9 à 12 caractérisée en ce que l'amino-acide est un

composé naturel ou synthétique, optiquement actif ou non, ou racémique.

14. Utilisation selon l'une quelconque des revendications 9 à 13 caractérisée en ce que $R^4$ représente un atome d'hydrogene, $R^3$ un radical aliphatique en $C_1$ à $C_{20}$, araliphatique en $C_7$ à $C_{20}$, substitué ou non, saturé ou non ; ou $R^3$ et $R^4$ sont reliés et forment avec l'atome d'azote un hétérocycle, avec 4 à 6 chaînons, saturé ou non, substitué ou non.

15. Utilisation selon l'une quelconque des revendications 9 à 14 caractérisée en ce que $R^3$ et/ou $R^4$ sont substitués par des groupes fonctionnels bloqués on non.

16. Utilisation selon l'une quelconque des revendications 9 à 15 caractérisée en ce que le groupe carboxylique de l'amino-acide est remplacé par un groupe sulfonique ou phosphorique.

17. Utilisation selon l'une quelconque des revendications 9 à 16 caractérisée en ce que l'amino-acide est sous forme d'un dérivé de l'acide.

18. Utilisation selon l'une quelconque des revendications 9 à 17 caractérisée en ce que l'amino-acide est la L-phénylalanine, la L-proline, la glycine, la L-tyrosine, la L-sérine, l'acide L-aspartique, le glycinate d'éthyle, la phénylglycine, la L-alanine, le tryptophane, l'ester benzylique de l'acide aspartique, l'histidine, le tosylate d'histidine, l'alanine, la valine, l'isoleucine, la leucine, la méthionine, l'ester benzylique de l'acide glutamique, l'éther benzylique de la tyrosine, de la sérine, de la thréonine, la nitroarginine, la benzyloxycarbonyl, lysine, l'acétomidométhyle cystéïne.

19. Utilisation selon l'une quelconque des revendications 9 à 18 caractérisée en ce que le milieu solvant est constitué d'un ou de plusieurs solvants inertes vis-à-vis des réactifs choisis parmi les solvants aliphatiques chlorés, les éthers cycliques ou non, l'acétone, la pyridine, l'acétonitrile, le diméthylformamide et les alcools.

20. Utilisation selon la revendication 19 caractérisée en ce que le milieu solvant contient de l'eau.

21. Utilisation selon la revendication 20 caractérisée en ce que le milieu solvant est un mélange dioxanne-eau dans le rapport 1 : 1.

22. Utilisation selon l'une quelconque des revendications 9 à 21 caractérisée en ce que l'agent de fixation d'acide est une base organique ou minérale.

23. Utilisation selon la revendication 22 caractérisée en ce que la base est choisie parmi l'hydroxyde de sodium ou de potassium, le carbonate ou le bicarbonate de sodium ou de potassium, l'oxyde de magnésium, les amines tertiaires.

24. Utilisation selon la revendication 23 caractérisée en ce que l'amine tertiaire est la pyridine ou la triéthylamine.

25. Utilisation selon l'une quelconque des revendications 9 à 24 caractérisée en ce que la réaction est effectuée à une température comprise entre 0° et 30° C.

26. Utilisation selon l'une quelconque des revendications 9 à 25 caractérisée en ce que l'on maintient constante la valeur du pH.

**Patentansprüche** für die Vertragsstaaten: BE, CH, DE, FR, GB, IT, LI, LU, NL, SE

1. Neue alpha-chlorierte Carbonate der Formel:

$R^1$ - O - C - O - CH - $CX_3$
ǁ |
O Cl

in der
X Fluor, Chlor oder Brom bedeutet und

$R^1$ von-CH - $CCl_3$ verschieden ist und
|
Cl

und einen gegebenenfalls substituierten, gegebenfalls gesättigten, primären, sekundären oder tertiären aliphatischen Rest mit 1 bis 20 Kohlenstoffatomen, arylaliphatischen Rest mit 7 bis 20 Kohlenstoffatomen oder zykloaliphatischen Rest mit 7 bis 20 Kohlenstoffatomen bedeutet, wobei die Substituenten von $R^1$ unter Halogen, Silicium enthaltenden Resten oder gegebenenfalls heterozyklischen Resten mit Stickstoff, Sauerstoff oder Schwefel gewählt werden.

2. Alpha-chlorierte Carbonate nach Anspruch 1,
dadurch gekennzeichnet,
daß in der Formel X Chlor ist.

3. Alpha-chlorierte Carbonate nacn Anspruch 1 oder 2,
dadurch gekennzeichnet,
daß in der Formel $R^1$ t-Butyl, 9-Fluorenylmethyl, 2,2,2-Trichlorethyl, Trimethylsilylethyl oder p-Nitrobenzyl ist.

4. Alpha-chloriertes Carbonat nach einem der vorstehenden Ansprüche,
dadurch gekennzeichnet,

daß es 1,2,2,2-Tetrachlorethyl-t-butylcarbonat ist.

5. Verfahren zur Herstellung der alpha-chlorierten Carbonate nach einem der vorstehenden Ansprüche, gekennzeichnet durch
Umsetzung einer Verbindung der Formel $R^1OH$ und eines alpha-chlorierten Chlorformiats der Formel:

$CX_3$ -CH - O - C - Cl
| ||
Cl O

wobei X und $R^1$ die oben angegebene Bedeutung haben, in einem Lösungsmittel bei einer Temperatur von -20° bis + 50°C in Gegenwart eines Säureakzeptors, der nach den beiden Ausgangsverbindungen zugegeben wird.

6. Verfahren nach Anspruch 5,
gekennzeichnet durch
Verwendung einer organischen oder anorganischen Base als Säureakzeptor.

7. Verfahren nach Anspruch 6,
gekennzeichnet durch
allmähliche Zugabe der Base.

8. Verfahren nach Anscpruch 6 oder 7,
gekennzeichnet durch
Verwendung von Pyridin oder Triethylamin als Base.

9. Verfahren nach einem der Ansprüche 5 bis 8,
gekennzeichnet durch
Verwendung mindestens eines gegenüber den gewählten Reaktionsteilnehmern inerten Lösungsmittels, ausgewählt unter chlorierten aliphatischen Lösungsmitteln, gegebenenfalls zyklischen Ethern, Ketonen, Nitrilen, Estern und aliphatischen oder aromatischen Kohlenwasserstoffen.

10. Verwendung der alpha-chlorierten Carbonate nach einem der Ansprüche 1 bis 4,
gekennzeichnet durch
Umsetzung des alpha-chlorierten Carbonats in einem Lösungsmittel in Gegenwart eines säurebindenden Mittels bei einer Temperatur von -5° bis 100°C und einer Amino-und/oder Iminocarbonsäure mit mindestens einem Wasserstoffatom auf der Amino- oder Iminogruppe der Formel

NH - $R^3$ - COOH
|
$R^4$

in der $R^3$ und $R^4$ unabhängig oder miteinander verbunden die üblichen Rest von natürlichen oder synthetischen Aminosäuren darstellen.

11. Verwendung nach Anspruch 10,
dadurch gekennzeichnet,
daß die Aminosäure eine natürliche oder synthetische, gegebenenfalls optisch aktive oder racemische Verbindung ist.

12. Verwendung nach Anspruch 10 oder 11,
dadurch gekennzeichnet,
daß in der Formel $R^4$ Wasserstoff ist, $R^3$ ein gegebenenfalls substituierter, gegebenenfalls gesättigter aliphatischer Rest mit 1 bis 20 Kohlenstoffatomen oder arylaliphatischer Rest mit 7 bis 20 Kohlenstoffatomen ist oder $R^3$ und $R^4$ miteinander verbunden sind und mit dem Stickstoffatom einen gegebenenfalls gesättigten, gegebenenfalls substituierten 4- bis 6-gliedrigen heterozyklischen Rest bilden.

13. Verwendung nach einem der Ansprüche 10 bis 12,
dadurch gekennzeichnet,
daß in der Formel $R^3$ und/oder $R^4$ mit gegebenenfalls blockierten funktionellen Gruppen substituiert sind.

14. Verwendung nach einem der Ansprüche 10 bis 13,
dadurch gekennzeichnet,
daß die Carboxylgruppe der Aminosäure durch eine Sulfo-oder Phosphonogruppe ersetzt ist.

15. Verwendung nach einem der Ansprüche 10 bis 14,
dadurch gekennzeichnet,
daß die Aminosäure in Form eines Säurederivats ist.

16. Verwendung nach einem der Ansprüche 10 bis 15,
dadurch gekennzeichnet,
daß die Aminosäure L-Phenylalanin, L-Prolin, Glycin, L-Tyrosin, L-Serin, L-Asparaginsäure, Ethylglycinat, Phenylglycin, L-Alanin, Tryptophan, Asparaginsäure-benzylester, Histidin, Histidin-tosylat, Alanin, Valin, Isoleucin, Leucin, Methionin, Glutaminsäure-benzylester, Tyrosin-, Serin- oder Threonin-benzyl -äther, Nitroarginin, Benzyloxycarbonyllysin oder Acetamidomethylcystein ist.

17. Verwendung nach einem der Anspruche 10 bis 16,
dadurch gekennzeichnet,
daß das Lösungsmittel aus mindestens einem gegenüber den gewählten Reaktionsteilnehmern inerten

Lösungsmittel besteht, ausgewählt unter chlorierten aliphatischen Lösungsmitteln, gegebenenfalls zyklischen Ethern, Aceton, Pyridin, Acetonitril, Dimethylformamid und Alkoholen.

18. Verwendung nach Anspruch 17,
dadurch gekennzeichnet,
daß das Lösungsmittel Wasser enthält.

18. Verwendung nach Anspruch 18,
dadurch gekennzeichnet,
daß das Lösungsmittel ein Dioxan-Wasser-Gemisch im Verhältnis 1 : 1 ist.

20. Verwendung nach einem der Ansprüche 10 bis 19,
dadurch gekennzeichnet,
daß das säurebindende Mittel eine organische oder anorganische Base ist.

21. Verwendung nach Anspruch 20,
dadurch gekennzeichnet,
daß die Base unter Natrium- oder Kaliumhydroxid, Natrium- oder Kaliumcarbonat oder -hydrogencarbonat, Magnesiumoxid und tertiären Aminen ausgewählt wird.

22. Verwendung nach Anspruch 21,
dadurch gekennzeichnet,
daß das tertiäre Amin Pyridin oder Triethylamin ist.

23. Verwendung nach einem der Ansprüche 10 bis 22,
dadurch gekennzeichnet,
daß die Reaktion bei einer Temperatur von 0° bis 30°C durchgeführt wird.

Verwendung nach einem der Ansprüche 10 bis 23,
dadurch gekennzeichnet,
daß ein konstanter pH-Wert eingehalten wird.

**Patentansprüche** für den Vertragsstaat: AT

1. Verfahren zur Herstellung von alpha-chlorierten Carbonaten der Formel

```
R1 - O C- O -   CH - CX3
       ||       |
       O        Cl
```

in der
X Fluor, Chlor oder Brom bedeutet und

```
R1 von -  CH - CCl3 verschieden ist und
          |
          Cl
```

einen ggf, substituierten, ggf. gesättigten, primären, sekundären oder tertiären aliphatischen Rest mit 1 bis 20 Kohlenstoffatomen, arylaliphatischen Rest mit 7 bis 20 Kohlenstoffatomen oder cycloaliphatisehen Rest mit 7 bis 20 Kohlenstoffatomen bedeutet, wobei die Substituenten von $R^1$ unter Halogenatomen, Silicium enthaltenden Resten oder ggf. heterocyclischen Resten mit Stickstoff, Sauerstoff oder Schwefel gewählt werden, gekennzeichnet durch Umsetzung einer Verbindung der Formel $R^1OH$ und eines alpha-chlorierten Chlorformiats der Formel

```
CX3 -  CH - O -  C - Cl
       |         ||
       Cl        O
```

wobei X und $R^1$ die oben angegebenen Bedeutungen haben, in einem Lösungsmittel bei einer Temperatur von -20 bis +50°C in Gegenwart eines Säureakzeptors, der nach den beiden Ausgangsverbindungen zugegeben wird.

2. Herstellungsverfahren nach Anspruch 1, dadurch gekennzeichnet, daß in der Formel X Chlor ist.

3. Herstellungsverfahren nach Anspruch 1 oder 2, dadurch gekennzeichnet, daß in der Formel $R^1$ t-Butyl, 9-Fluorenylmethyl, 2,2,2,-Trichlorethyl, Trimethylsilylethyl oder p-Nitrobenzyl ist.

4. Herstellungsverfahren nach Anspruch 1, dadurch gekennzeichnet, daß das hergestellte Carbonat 1,2,2,2-Tetrachlorethyl-t-butylcarbonat ist.

5. Verfahren nach einem der Vorstehenden Ansprüche, gekennzeichnet durch Verwendung einer organischen oder anorganischen Base als Säureakzeptor.

6. Verfahren nach Anspruc 5, gekennzeichnet durch ällmähliche Zugabe der Base.

7. Verfahren nach Anspruch 5 oder 6, gekennzeichnet durch Verwendung von Pyridin oder Triethylalmin als

Base.

8. Verfahren nach einem der vorstehenden Ansprüche, gekennzeichnet durch Verwendung mindestens eines gegenüber den gewählten Reaktionsteilnehmern inerten Lösungsmittels, ausgewählt unter chlorierten aliphatischen Lösungsmitteln, ggf. cyclischen Ethern, Ketonen, Nitrilen, Estern und aliphatischen oder aromatischen Kohlenwasserstoffen.

9. Verwendung der alpha-chlorierten Carbonate der Formel

$R^1$ - O - C - O - CH - $CX_3$
‖ |
O Cl

in der

X Fluor, Chlor oder Brom bedeutet und

$R^1$ von - CH - $CCl_3$ verschieden ist und
|
Cl

einen ggf. substituierten, ggf. gesättigten, primären, sekundären oder tertiären aliphatischen Rest mit 1 bis 20 Kohlenstoffatomen, arylaliphatischen Rest mit 7 bis 20 Kohlenstoffatomen oder cycloaliphatischen Rest mit 7 bis 20 Kohlenstoffatomen bedeutet, wobei die Substituenten von $R^1$ unter Halogenatomen, Silicium enthaltenden Resten, ggf. heterocyclischen Resten mit Stickstoff, Sauerstoff oder Schwefel gewählt werden, gekennzeichnet durch Umsetzung des alpha-chlorierten Carbonats in einem Lösungsmittel in Gegenwart eines säurebindenden Mittels bei einer Temperatur von -5 bis 100°C und einer Amino- und/oder Iminocarbonsäure mit mindestens einem Wasserstoffatom an der Amino-oder Iminogruppe der Formel

NH - $R^3$ - COOH
|
$R^4$

in der $R^3$ und $R^4$ unabhängig oder miteinander verbunden die üblichen Reste von natürlichen oder synthetischen Aminosäuren darstellen.

10. Verwendung nach Anspruch 9, dadurch gekennzeich net, daß in der Formel der alpha-chlorierten Carbonate X Chlor ist.

11. Verwendung nach Anspruch 9 oder 10, dadurch gekennzeichnet, daß in der Formel der alpha-chlorierten Carbonate $R^1$ t-Butyl, 9-Fluorenylmetnyl, 2,2,2-Tri-chlorethyl, Trimethylsilyletnyl oder p-Nitrobenzyl ist.

12. Verwendung nach Anspruch 9, dadurch gekennzeichnet, daß das alpha-chlorierte Carbonat 1,2,2,2-Tetrachlorethyl-t-butylcarbonat ist.

13. Verwendung nach einem der Ansprüche 9 bis 12, dadurch gekennzeichnet, daß die Aminosäure eine natürliche oder Synthetische, ggf. optisch aktive oder racemische Verhindung ist.

14. Verwendung nach einem der Anspruche 9 bis 13, dadurch gekennzeichnet, daß in der Formel $R^4$ Wasserstoff ist, $R^3$ ein ggf. substituierter, ggf. gesättigter, aliphatischer Rest mit 1 bis 20 Kohlenstoffatomen oder arylaliphatischer Rest mit 7 bis 20 Kohlenstoffatomen ist, oder $R^3$ und $R^4$ miteinander verbunden sind und mit dem Stickstoffatom einen ggf. gesättigten, ggf. substituierten 4- bis 6-gliedrigen heterocyclischen Rest bilden.

15. Verwendung nach einem der Ansprüche 9 bis 14, dadurch gekennzeichnet, daß in der Formel $R^3$ und/oder $R^4$ mit ggf. blockierten funktionellen Gruppen substituiert sind.

16. Verwendung nach einem der Ansprüche 9 bis 15, dadurch gekennzeichnet, daß die Carboxylgruppe der Aminosäure durch eine Sulfo- oder Phosphonogruppe ersetzt ist.

17. Verwendung nach einem der Ansprüche 9 bis 16, dadurch gekennzeichnet, daß die Aminosäure in Form eines Säurederivats ist.

18. Verwendung nach einem der Ansprüche 9 bis 17, dadurch gekennzeichnet, daß die Aminosäure L-Phenylalanin, L-Prolin, Glycin, L-Tyrosin, L-Serin, L-Asparaginsäure, Ethylglycinat, Phenylglycin, L-Alanin, Tryptophan, Asparaginsäure-benzylester, Histidin, Histidin-tosylat, Alanin, Valin, Isoleucin, Leucin, Methlonin, Glutaminsäure-benzylexter, Tyrosin-, Serin- oder Threonin-benzylether, Nitroarginin, Benzyloxycarbonyllysin oder Acetamidomethylcystein ist.

19. Verwendung nach einem der Ansprüche 9 bis 18, dadurch gekennzeichnet, daß das Lösungsmittel aus mindestens einem gegenüber den gewählten Reaktionsteilnehmern inerten Lösungsmittel besteht, ausgewählt unter chlorierten aliphatischen Lösungsmitteln, ggf. cyclischen Ethern, Aceton, Pyridin, Acetonitril, Dimethylformamid und Alkoholen.

20. Verwendung nach Anspruch 19, dadurch gekennzeichnet, daß das Lösungsmittel Wasser enthalt.

21. Verwendung nach Anspruch 20, dadurch gekennzeichnet, daß das Lösungsmittel ein Dioxan-Wasser-Gemisch im Verhältnis 1 : 1 ist.

22. Verwendung nach einem der Ansprüche 9 bis 21, dadurch gekennzeichnet, daß das säurebindende Mittel eine organische oder anorganische Base ist.

23. Verwendung nach Anspruch 22, dadurch gekennzeichnet, daß die Base unter Natrium- oder Kaliumhydroxid-Natrium- oder Kaliumcarbonat oder -hydrogencarbonat-Magnesiumoxid und tertiären Aminen ausgewählt wird.

24. Verwendung nach Anspruch 23, dadurch gekennzeichnet, daß das tertiäre Amin pyridin oder Triethylamin ist.

25. Verwendung nach einem der Ansprüche 9 bis 24, dadurch gekennzeichnet, daß die Reaktion bei einer Temperatur von 0 bis 30° C durchgeführt wird.

26. Verwendung nach einem dar Ansprüche 9 bis 25, dadurch gekennzeichnet, daß ein konstanter pH-Wert eingehalten wird.

**Claims** for the contracting States: BE, CH, DE, FR, GB, IT, LI, LU, NL, SE

1. New alpha-chlorinated carbonates characterized in that they have the formula:

```
R1 - O- C -  O - CH - CX3
        ||      |
        O       Cl
```

in which X represents a fluorine, chlorine or bromine atom and $R^1$ is other than the group

```
 - CH - CCl3 and
   |
   Cl
```

represents:

a substituted or unsubstituted, saturated or unsaturated $C_1$ to $C_{20}$ aliphatic or $C_7$ to $C_{20}$ araliphatic which may be primary, secondary or tertiary or $C_7$ to $C_{20}$ alicyclic radical, the substituents of $R^1$ being chosen from amongst halogen atoms, groups containing silicon, and heterocyclic or non-heterocyclic groups containing nitrogen, oxygen or sulphur.

2. Alpha-chlorinated carbonates according to Claim 1, charcterized in that X is chlorine.

3. Alpha-chlorinated carbonates according to any one of the preceding claims, characterized in that $R^1$ is the tert-butyl, 9-fluorenylmethyl, 2,2,2-trichloroethyl, trimethylsilylethyl or para-nitrobenzyl group.

4. Alpha-chlorinated carbonate according to any one of the preceding claims, characterized in that it is 1,2,2,2-tetrachloroethyl tert-butyl carbonate.

5. Process for the preparation of alpha-chlorinated carbonates according to any one of the preceding claims, characterized in that a compound of formula $R^1OH$ is reacted with an alpha-chlorinated chloroformate of formula:

```
CX3 -CH - O -  C - Cl
     |         ||
     Cl        O
```

X and $R^1$ having the above meanings, in a solvent medium at a temperature from -20° to +50° C in the presence of an acid-accepting agent which is added following the two starting compounds.

6. Process according to Claim 5, characterized in that the acid-accepting agent is an organic or inorganic base.

7. Process according to Claim 6, characterized in that the base is added gradually.

8. Process according to Claim 6 or 7, characterized in that the base is pyridine or triethylamine.

9. Process according to any one of Claims 5 to 8, characterized in that the solvent medium consists of one or more solvents which are inert towards the reagents, chosen from amongst chlorinated aliphatic solvents, cyclic or non-cyclic ethers, ketones, nitriles, esters and aliphatic or aromatic hydrocarbons.

10. Use of the alphachlorinated carbonates according to any one of Claims 1 to 4, characterized in that the alpha-chlorinated carbonate is reacted, in a solvent medium in the presence of an acid-binding agent and at a temperature of between -5° and 100° C, with an amino- and/or iminocarboxylic acid containing at least one hydrogen atom bound to the amino or imino group, of formula

```
NH - R3 - COOH
|
R4
```

in which $R^3$ and $R^4$, independently or linked together, represent the usual radicals of natural or synthetic amino acids.

11. Use according to Claim 10, characterized in that the amino acid is a natural or synthetic compound which

may or may not be optically active or may be racemic.

12. Use according to Claim 10 or 11, characterized in that $R^4$ represents a hydrogen atom, $R^3$ a $C_1$ to $C_{20}$ aliphatic or $C_7$ to $C_{20}$ araliphatic radical which may be substituted or unsubstituted, saturated or unsaturated; or $R^3$ and $R^4$ are linked together and form, with the nitrogen atom, a saturated or unsaturated, substituted or unsubstituted 4- to 6-membered heterocycle.

13. Use according to any one of Claims 10 to 12, characterized in that $R^3$ and/or $R^4$ are substituted with functional groups which may blocked or otherwise.

14. Use according to any one of Claims 10 to 13, characterized in that the carboxyl group of the amino acid is replaced by a sulphonic or phosphoric group.

15. Use according to any one of Claims 10 to 14, characterized in that the amino acid is in the form of an acid derivative.

16. Use according to any one of Claims 10 to 15, characterized in that the amino acid is L-phenylalanine, L-proline, glycine, L-tyrosine, L-serine, L-aspartic acid, ethyl glycinate, phenylglycine, L-alanine, L-tryptophan, benzyl ester of aspartic acid, histidine, histidine tosylate, alanine, valine, isoleucine, leucine, methionine, benzyl ester of glutamic acid, benzyl ether of tyrosine, of serine and of threonine, nitroarginine, benzyloxycarbonyllysine or acetamidomethylcysteine.

17. Use according to any one of Claims 10 to 16, characterized in that the solvent medium consists of one or more solvents which are inert towards the reagents, chosen from amongst chlorinated aliphatic solvents, cyclic or non-cyclic ethers, acetone, pyridine, acetonitrile, dimethylformamide and alcohols.

18. Use according to Claim 17, characterized in that the solvent medium contains water.

19. Use according to Claim 18, characterized in that the solvent medium is a dioxane-water mixture in the ratio 1 : 1.

20. Use according to any one of Claims 10 to 19, characterized in that the acid-binding agent is an organic or inorganic base.

21. Use according to Claim 20, characterized in that trh base is chosen from amongst sodium or potassium hydroxide, sodium or potassium carbonate or bicarbonate, magnesium oxide and tertiary amines.

22. Use according to Claim 21, characterized in that the tertiary amine is pyridine or triethylamine.

23. Use according to any one of Claims 10 to 22, characterized in that the reaction is carried out at a temperature of between 0° and 30°C.

24. Use according to any one of Claims 10 to 23, characterized in that the pH value is maintained constant.

**Claims** for the contracting State: AT

1. Process for preparing alpha-chloro carbonates of the formula:

$R^1$ - O -C - O - CH - $CX_3$
ǁ | 
O Cl

in which X represents a fluorine, chlorine or bromine atom and $R^1$ is other than the group

- CH-$CCl_3$
|
Cl

and represents:

a substituted or unsubstituted, saturated or unsaturated $C_1$ to $C_{20}$ aliphatic or $C_7$ to $C_{20}$ araliphatic radical which may be primary, secondary or tertiary, or a $C_7$ to $C_{20}$ alicyclic radical, the substituents of $R^1$ being chosen from halogen atoms, groups containing silicon, and heterocyclic or nonheterocyclic groups containing nitrogen, oxygen or sulphur atoms, characterized in that a compound of formula $R^1OH$ is reacted with an alpha-chloro chloroformate of formula:

$CX_3$ -CH - O - C - Cl
| ǁ
CL O

X and $R^1$ having the above meanings, in a solvent medium at a temperature of -20° to +50°C in the presence of an acid acceptor agent which is added following the two starting compounds.

2. Preparation process according to Claim 1, characterized in that X is chlorine.

3. Preparation process according to Claim 1 or 2, characterized in that $R^1$ is the tert-butyl, 9-fluorenyl-methyl, 2,2,2-trichloroethyl, trimethylsilylethyl or para-nitrobenzyl group.

4. Preparation process according to Claim 1, characterized in that the carbonate prepared is 1,2,2,2-tetrachloroethyl tert-butyl carbonate.

5. Process according to any one of the preceding claims, characterized in that the acid acceptor agent is an

organic or inorganic base.

6. Process according to Claim 5, characterized in that the base is added gradually.

7. Process according to Claim 5 or 6, characterized in that the base is pyridine or triethylamine.

8. Process according to any one of the preceding claims, characterized in that the solvent medium consists of one or more solvents which are inert towards the reactants, chosen from chlorinated alphatic solvents, cyclic or acyclic ethers, ketones, nitriles, esters and aliphatic or aromatic hydrocarbons.

9. Use of the alpha-chloro carbonates of formula:

```
R1 - O -C - O -  CH - CX3
        ||       |
        O        Cl
```

in which X represents a fluorine, chlorine or bromine atom and $R^1$ is other than the group

```
- CH-CCl3
  |
  Cl
```

and represents:

a substituted or unsubstituted, saturated or unsaturated $C_1$ to $C_{20}$ aliphatic or $C_7$ to $C_{20}$ araliphatic radical which may be primary, secondary or tertiary, or a $C_7$ to $C_{20}$ alicyclic radical, the substituents of $R^1$ being chosen from halogen atoms, groups containing silicon, and heterocyclic or nonheterocyclic groups containing nitrogen, oxygen or sulphur atoms, characterized in that the alpha-chloro carbonate is reacted in a solvent medium in the presence of an acid-binding agent and at a temperature of between -5° and 100°C with an amino- and/or iminocarboxylic acid containing at least one hydrogen atom bonded to the amino or imino group, of formula

```
NH - R3 - COOH
|
R4
```

in which $R^3$ and $R^4$ independently or jointly represent the usual radicals of natural or synthetic amino acids.

10. Use according to Claim 9, characterized in that in the formula of the alpha-chloro carbonates X represents chlorine.

11. Use according to Claim 9 or 10, characterized in that the group $R^1$ in the alpha-chloro carbonates represents the tert-butyl, 9-fluorenylmethyl, 2,2,2-trichloroethyl, trimethylsilyl or para-nitrobenzyl group.

12. Use according to Claim 9, characterized in that the alpha chloro carbonate is 1,2,2,2-tetrachloroethyl tert-butyl carbonate.

13. Use according to any one of Claims 9 to 12, characterized in that the amino acid is a natural or synthetic, inactive or optically active or racemic compound.

14. Use according to any one of Claims 9 to 13, characterized in that $R^4$ represents a hydrogen atom and $R^3$ a $C_1$ to $C_{20}$ aliphatic or $C_7$ to $C_{20}$ araliphatic radical which may be substituted or unsubstituted, and saturated or unsaturated; or $R^3$ and $R^4$ are linked and form with the nitrogen atom a saturated or unsaturated, substituted or unsubstituted 4- to 6-membered heterocyclic ring.

15. Use according to any one of Claims 9 to 14, characterized in that $R^3$ and/or $R^4$ are substituted by functional groups which may be blocked or otherwise.

16. Use according to any one of Claims 9 to 15, characterized in that the carboxyl group of the amino acid is replaced by a sulphonic or phosphoric group.

17. Use according to any one of Claims 9 to 16, characterized in that the amino acid is in the form of an acid derivative.

18. Use according to any one of Claims 9 to 17, characterized in that the amino acid is L-phenylalanine, L-proline, glycine, L-tyrosine, L-serine, L-aspartic acid, ethyl glycinate, phenylglycine, L-alanine, tryptophan, benzyl ester of aspartic acid, histidine, histidine tosylate, alanine, valine, isoleucine, leucine, methionine, benzyl ester of glutamic acid, benzyl ether of tyrosine, of serine and of threonine, nitroarginine, benzyloxycarbonyl, lysine or acetamidomethylcysteine.

19. Use according to any one of Claims 9 to 18, characterized in that the solvent medium consists of one or more solvents which are inert towards the reactants, chosen from chlorinated al iphatic solvents, cyclic or acyclic ethers, acetone, pyridine, acetonitrile, dimethylformamide and alcohols.

20. Use according to Claim 19, characterized in that the solvent medium contains water.

21. Use according to Claim 20, characterized in that the solvent medium is a dioxane-water mixture in the ratio 1 : 1.

22. Use according to any one of Claims 9 to 21, characterized in that the acid-binding agent is an organic or inorganic base.

23. Use according to Claim 22, characterized in that the base is chosen from sodium or potassium hydroxide, sodium or potassium carbonate or bicarbonate, magnesium oxide or tertiary amines.

24. Use according to Claim 23, characterized in that the tertiary amine is pyridine or triethylamine.

25. Use according to any one of Claims 9 to 24, characterized in that the reaction is carried out at a temperature of between 0° and 30° C.

26. Use according to any one of Claims 9 to 25, characterized in that the pH value is maintained constant.